# EUROPEAN PATENT SPECIFICATION

(11) **EP 2 473 626 B1**
(45) Date of publication and mention of the grant of the patent: **29.01.2014**
(21) Application number: 10760024.9
(22) Date of filing: 31.08.2010
(51) Int. Cl.: C12Q 1/68

(54) **ARRAY-BASED METHOD FOR DETECTION OF COPY NUMBER VARIATIONS IN THE HLA LOCUS FOR THE GENETIC DETERMINATION OF SUSCEPTIBILITY OF DEVELOPMENT OF VENOUS MALFORMATIONS IN THE EXTRACRANIAL SEGMENTS OF THE CEREBROSPINAL VEINS AND ARRAY THEREOF**
ARRAYBASIERTES VERFAHREN ZUR ERKENNUNG VON VERVIELFÄLTIGUNGSZAHLVARIATIONEN BEIM HLA-LOCUS ZUR GENETISCHEN BESTIMMUNG DER PRÄDISPOSITION FÜR VENÖSE MALFORMATIONEN IN DEN EXTRAKRANIELLEN SEGMENTEN DER ZEREBROSPINALEN VENEN UND ARRAY DAFÜR
PROCÉDÉ MATRICIEL DE DÉTECTION DES VARIATIONS DU NOMBRE DE COPIES DANS LE LOCUS HLA POUR LA DÉTERMINATION GÉNÉTIQUE DU RISQUE DE DÉVELOPPEMENT DE MALFORMATIONS VEINEUSES DANS LES SEGMENTS EXTRACRÂNIENS DES VEINES CÉPHALO-RACHIDIENNES ET ARRAY CORRESPONDANT

(30) Priority: 01.09.2009 IT TO20090672
(43) Date of publication of application: 11.07.2012
(73) Proprietor: London Equitable Limited in its capacity as Trustee of the Think Tank Trust, Mayfair London W1J 7SX (GB)
(72) Inventor: ZAMBONI, Paolo, I-44100 Ferrara (IT); FERLINI, Alessandra, I-44100 Ferrara (IT); BOVOLENTA, Matteo, I-44100 Ferrara (IT)
(74) Representative: Freyria Fava, Cristina
(86) International application number: PCT/IB2010/053901
(87) International publication number: WO 2011/027292

(56) References cited:
- WO-A2-2008/008487
- WO-A2-2008/075977
- US-A1- 2006 115 826
- COMABELLA MANUEL ET AL: "Identification of a Novel Risk Locus for Multiple Sclerosis at 13q31.3 by a Pooled Genome-Wide Scan of 500,000 Single Nucleotide Polymorphisms", PLOS ONE, vol. 3, no. 10, E3490, October 2008 (2008-10), pages 1-9, XP002566685, ISSN: 1932-6203
- KASUGAI YUMIKO ET AL: "Identification of CCND3 and BYSL as candidate targets for the 6p21 amplification in diffuse large B-cell lymphoma.", CLINICAL CANCER RESEARCH : AN OFFICIAL JOURNAL OF THE AMERICAN ASSOCIATION FOR CANCER RESEARCH, vol. 11, no. 23, 1 December 2005 (2005-12-01), pages 8265-8272, XP002566686, ISSN: 1078-0432
- FITEN PIERRE ET AL: "Microsatellite polymorphisms in the gene promoter of monocyte chemotactic protein-3 and analysis of the association between monocyte chemotactic protein-3 alleles and multiple sclerosis development", JOURNAL OF NEUROIMMUNOLOGY, vol. 95, no. 1-2, 1 March 1999 (1999-03-01), pages 195-201, XP002566687, ISSN: 0165-5728
- SANUS ET AL: "Cerebral cavernomas and human leukocyte antigens: preliminary clinical results", SURGICAL NEUROLOGY, LITTLE, BROWN AND CO., BOSTON, MA, US, vol. 68, no. 2, 24 July 2007 (2007-07-24), pages 164-166, XP022165895, ISSN: 0090-3019

## Description

### Field of the invention

This disclosure concerns a Array-based method for detection of Copy number variations in the HLA locus for the genetic determination of susceptibility of development of venous malformations in the extracranial segments of the cerebrospinal veins associated with multiple sclerosis. More specifically, the present disclosure concerns a genetic diagnostic method for the determination of the genotype-phenotype correlation risk of development of venous malformations associated with multiple sclerosis.

### Background of the invention

Multiple sclerosis is the most common neurological disease in young adult population catalogued into neurodegenerative disorders of unknown etiology. Inflammatory, infective, and autoimmune causes have been proposed to have a pathogenic role in this disease, although the link between these factors and the disease etiology remains to be elucidated.

From the genetic point of view, studies on twins and siblings suggest that susceptibility genes may play a role in this disease. Multiple sclerosis has, in fact, a clinically significant heritable component.

A genomewide association study was carried out in order to identify alleles associated with the risk of multiple sclerosis [7]. A transmission disequilibrium test of 334,923 single-nucleotide polymorphisms (SNPs) in 931 family trios revealed 49 SNPs having an association with multiple sclerosis. Alleles of IL2RA and IL7RA and those in the HLA locus are identified as heritable risk factors for multiple sclerosis. However, the detection of susceptibility loci is an important starting point, but does not clarify what are exactly the genes involved in the transmission of the disease and through what molecular mechanisms. Moreover, a correlation between genotype variation and phenotype phenomena has not been demonstrated. For such a reason, susceptibility loci need to be finely mapped and also correlated with the function of the component genes.

Candidate-gene studies and whole genome association studies as well as copy number variations (CNVs) detection on single nucleotide polymorphisms (SNPs) based arrays involving more than hundred thousand markers have been performed and identified several susceptibility loci in the human genome, being the HLA locus on 6p21.32 the more confidently associated locus [1-6]. A few other possible susceptibility loci have been described although of uncertain statistical significance [7,8].

When using single nucleotide polymorphisms (SNPs) based arrays and even when controls are accurately randomised, undetectable errors may occur especially linked to the population geographical origins, to the known differences in SNPs density, depending on the various human chromosomes or even genomic regions involved. These errors may inflate the apparently significant differences between patients and controls (genomic inflation) generating false positive or false negative, and finally hampering a true recognition of the associated loci [8].

In order to overcome this "potential" trouble, an enormous number of individuals have to be analysed as recommend by the Wellcome Trust Case Control Consortium and as recently reported [8], to get unbiased data and to replicate the associations in the identified loci. However, the Authors themselves conclude that functional studies are required.

### Summary of the invention

Taking into account these premises, the need is therefore felt for improved solutions enabling reliable detection of genetic predisposition factors of patients to development of venous malformations associated with multiple sclerosis.

The object of this disclosure is providing such improved solutions.

According to the invention, the above object is achieved thanks to the subject matter recalled specifically in the ensuing claims, which are understood as forming an integral part of this disclosure.

An embodiment of the present disclosure provides a method for *in vitro* diagnosis of risk of development of at least one extra cranial cerebrospinal venous malformation in a patient, comprising the step of detecting the global number of extragenic copy number variations (CNVs) in the HLA-DRA locus of chromosome 6p21, wherein the number of extragenic CNVs correlates with said risk.

The present disclosure discloses a kit for performing such a diagnostic method. More specifically, the kit comprises CGH probes covering the HLA-DRA locus region for the detection of CNVs in chromosome 6p21 in genomic DNA of patients.

A further embodiment of the present disclosure concerns a CGH array entirely covering the HLA-DRA locus region for the detection of CNVs in chromosome 6p21 in genomic DNA of patients.

The data herein disclosed demonstrate a significant correlation between the number of CNVs found in the HLA-DRA region and the number of venous malformations, more specifically of chronic cerebrospinal venous malformations, identified in patients. This disclosure demonstrates that the number of multiple polymorphic CNVs in the HLA locus identified are determinants possibly involved in the phenotypic manifestation to this novel venous malformations/multiple sclerosis association.

### Brief description of the drawings

The invention will now be described, by way of example only, with reference to the enclosed figures of drawing, wherein:
- **Figure 1A** **and** **1B****:** Genomic distribution of the Known CNVs among the patients studied along the HLA-DRA locus. In the graph are reported the starting nucleotide for each CNV. In white are highlighted the regions of the HLA genes.
- **Figure 2****:** Exemplification of stenosing venous malformation associated to MS. A) Significant stenosis (arrow) of the left internal jugular vein (L IJV). B) Membranous obstruction of the outlet of the Azygous vein (o AZY) into the superior vena cava (SVC).
- **Figure 3 to 5****:** Linear regression analysis. A significant correlation between the number CNVs (figure 3), intragenic CNVs (figure. 4) and extragenic CNVs (figure 5) and the number of venous malformations detected by the means of selective venography was found (r=0.53, r2=0.28, p<0.05).
- **Figure 6****:** Number of total (A), intragenic (B) and extragenic (C) known CNVs reported in the Database of Genomic Variants per patient.
- **Figure 7****:** Functional links of the known genes within the HLA locus networking on neurodegenration, multiple sclerosis and immunity disorders (A) and angiogenesis and venous formation pathways (B).- **Figure 8****:** The CGH profile identified in patient PF as example. The multiple perturbation of the genomic area is well apparent.

### Detailed description of the invention

In the following description, numerous specific details are given to provide a thorough understanding of embodiments. The embodiments can be practiced-without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The headings provided herein are for convenience only and do not interpret the scope or meaning of the embodiments.

The present inventors to investigate the occurrence of copy number variations (CNVs) underlining genome unbalances in the major locus (HLA, chromosome 6p21) associated with multiple sclerosis (MS) recur to a comparative genomic hybridisation (CGH) array in order to overcome the inevitable errors associated with the SNPs-based arrays.

It is now well recognised that copy number variations (CNVs) typically ranging from 1 kb to several Mb do contribute to genetic variations and disease susceptibility. CNVs account for more nucleotide variations between individuals; in addition, the functional meaning of CNVs might be more immediate, especially for those located within genes, regulatory regions or known imprinted regions, since the possible consequence of the genome unbalance(s) may be interpretable in a more straight forward way.

The advantage to search for CNVs by the CGH technique is the possibility to directly correlate the known, validated CNVs with a possible function, both related to the specific gene unbalanced and to other genomic regions with copy variations. In fact, CGH approach allows to finely mapping the identified CNVs in non-genic regions, possibly correlated to genes regulatory function, epigenetic changes or other noncoding functions.

The development of robust high throughput platforms based on comparative genomic hybridisation (CGH) capable of identifying thousand genomic variations has greatly improved the research in this direction, as recently demonstrated in a major neurodegenerative disorder, the amiotrophic lateral sclerosis, in which non-polymorphic sub-microscopic duplications and deletions seem to be frequent in sporadic cases.

The present inventors designed a locus-specific CGH array in order to explore the occurrence of CNVs in the HLA-DRA locus region (6,899,999 bp; chromosome 6p21: 29,900,001-36,800,000 bp) in 15 patients with the peculiar association of chronic cerebrospinal venous insufficiency (CCSVI) and MS phenotype.

The present inventors already described the peculiar association of chronic cerebrospinal venous insufficiency (CCSVI) in patients with Ms in WO 2009 107 152. In the application the inventors demonstrated that CCSVI is due to stenosing venous malformations (VM) affecting the azygous and the jugular veins, leading to significant anomalies in cerebral venous outflow haemodynamics. The hampered cerebral venous return in consequence of the extracranial venous malformations is peculiar to MS, and was not found in a miscellaneous of patients affected by other neurodegenerative disorders, such as Parkinson's, Alzheimer's, amyotrophic lateral sclerosis.

In total patients showed 322 CNVs of which 225 extragenic and 97 intragenic. The present inventors identified 234 known polymorphic CNVs in the 15 patients having such a "plus" phenotype (i.e. VM-CCSVI and MS phenotype). Looking at the distribution of the polymorphic CNVs identified in these patients, the present inventors observed a peak of CNVs number within the HLA region. Outside this specific region however, the number of CNVs per patients remains high, though with variable distribution.

Interesting, the overall number of CNVs showed a correlation with the number of stenosing VM as demonstrated by venography in the extracranial segments of the cerebrospinal veins, with a trend toward significance.

The contribution to the correlation is certainly due to the extragenic CNVs, being significantly correlated to the number of stenosing VM (r=0.53, r2=0.28, p<0.05).

This result suggests that the contribution of extragenic CNVs in the development of the associated VM is related to their regulatory action in the process of angiogenesis.

The region studied contains 211 known genes. Using the functional bioinformatics tool the present inventors identify many genes interacting in both neurodegenerative and angiogenesis circuits. Notably, HSPA1L, HSP1A and HSP1B and the HLADQ2 genes network on both pathways. Heat-shock proteins (HSPs) represent a group of regulatory proteins involved in a variety of processes, including immunity and angiogenesis. In particular, HSPA1L expression is modulated by ETS1 transcription factor and by SP100 a nuclear autoimmune antigen. Interestingly, genes negatively regulated by ETS1 and up-regulated by SP100, as HSPA1L, have anti-migratory or anti-angiogenic properties.

MS has a very well known major heritable component since its susceptibility is associated with the MHC class II region, especially HLA-DRB5*0101-HLA-DRB1*1501-HLA-DQA1*0102-HLADQB1*0602 haplotypes, which dominate genetic contribution to MS risk [20]. Interestingly HLADQA2 is known to be involved with pro-inflammatory CD4(+) T-cell-mediated autoimmune diseases, such as MS and type 1 diabetes [21]. CD4 is also a very well known inhibitor of tumor angiogenesis [22], so supporting a link between the two pathways. The interpretation of the pathway interaction is obviously complex, but it suggests biological and functional links among these genes as well as, intriguingly, between angiogenesis and immunity.

Consistently with the general meaning of extragenic CNVs, putatively involved in gene expression regulation, this finding is interesting, since it supports the possibility that the number of structural variations laying within regulatory regions may represent a genomic "perturbation" increasing susceptibility for VM phenotype associated with MS the present inventors described.

Regarding specific candidate genes which expression could be potentially disturbed by genomic unbalances or "perturbation", the pathways analysis suggests that genes involved in angiogenesis and immunity are the more interesting proteins.

### Methods

### Subjects

Fifteen patients affected by the relapsing-remitting clinical course of MS diagnosed according to the revised Mc Donald criteria as disclosed in [11] entered the study. The present inventors determined the expanded disability disease score (EDSS) as disclosed in [12], as well as the multiple sclerosis severity score (MS-SS) as disclosed in [13,14].

The Kurtzke Expanded Disability Status Scale (EDSS) is a method of quantifying disability in multiple sclerosis. The EDSS categorizes a person's level of disability. EDSS scores range from 0-10, with higher scores indicating more severe disability. The MS-SS is a relationship between the EDSS and the disease duration, being the MS clinical course more aggressive in accordance with the time in which a certain score has been reached.

In the present population MS was associated with CCSVI venous malformation documented by a sequential Colour-Doppler/selective venography protocol as disclosed in the international patent application No. PCT/IT2008/000129 as well as in [9]. The clinical and demographic characteristics of the selected patients are given in the Table 1. Patients signed an informed consent. Detailed information about each of the 15 patients is provided in table 2.

**Table 1.**

| Parameters | Multiple sclerosis relapsing remitting n° 15 |
|---|---|
| | Median |
| | (IR) |
| Age, years | 36 |
| | (12) |
| Sex M/F | 7/8 |
| Disease duration, years | 6 |
| | (9) |
| EDSS | 1.5 |
| | (1) |
| MS-SS | 2.6 |
| | (3.8) |
| Number of VM | 2 |
| | (1) |
| Total CNVs | 23 |
| | (13) |

**Table 2.**

| **PATIENT CODE** | **SEX** | **AGE** | **N° OF VM** | **MS DISEASE DURATION YEARS** | **EDSS** | **MS-SS** | **Number of CNVs** |
|---|---|---|---|---|---|---|---|
| **PB** | F | 36 | 2 | 15 | 7 | 8,17 | 8 |
| **RM** | M | 31 | 3 | 3 | 1,5 | 3,34 | 31 |
| **DD** | M | 46 | 4 | 3 | 2 | 4,82 | 39 |
| **GE** | M | 46 | 2 | 6 | 1 | 1,13 | 20 |
| **BL** | M | 40 | 3 | 14 | 1,5 | 1,03 | 18 |
| **CC** | M | 32 | 3 | 4 | 1 | 1,45 | 14 |
| **FR** | M | 46 | 3 | 12 | 1 | 0,64 | 22 |
| **PF** | F | 27 | 2 | 4 | 1 | 1,45 | 12 |
| **CM** | F | 46 | 2 | 15 | 4 | 5,09 | 23 |
| **MU** | M | 40 | 2 | 9 | 1 | 0,88 | 10 |
| **CF** | F | 30 | 2 | 5 | 1,5 | 2,6 | 29 |
| **HH** | F | 36 | 3 | 3 | 1,5 | 3,34 | 15 |
| **LS** | F | 25 | 4 | 3 | 2 | 4,82 | 20 |
| **VF** | F | 36 | 2 | 6 | 0,5 | 0,25 | 12 |
| **MC** | F | 37 | 2 | 3 | 1,5 | 3,34 | 52 |

DNA from the 15 patients was extracted by protocol recommended by Agilent using the Qiagen DNeasy Blood & Tissue Kit. DNA from the 15 patients was extracted by protocol recommended by Agilent. Highly concentrated DNA was checked with a Nanodrop for quality (260/280 ratio =1.8 and 260/230 ratio =2.0). DNA integrity was evaluated on agarose gel at 1% in TBE 1X.

### Statistical Analysis

Clinical data.are given as median and interquartile range. Genotype-phenotype correlations were analyzed by the means of linear regression analysis, including evaluation of slope, X and Y Intercept, followed by Run Test. P values < 0.05 were considered to be significant.

### Microarray design, hybridization and data analysis

MS-CGH microarray design was carried out using the web based Agilent eArray database version 5.4 (Agilent Technologies, Santa Clara, CA) [16]. The high density aCGH search function within eArray was used to turn the genomic region chr6: 29,900,001-36,800,000 (March 2006 human reference sequence, NCBI Build 36.1, hg18;) into a probe set by selecting the maximum number of exonic, intronic and intragenic 60mer oligonucleotide CGH probes available in the database. This probe set included 43102 probes that were used to reach the array format of 4 x 44 K, creating four identical 44 K arrays on a single slide for simultaneous analysis of four different samples.

This platform called MS-CGH is a High-Density microarray with a resolution of one probe every 160 bp which allow the rapid determination of the molecular profile identifying the presence of copy number variations (CNVs) in heterozygosity or in homozygosity in the genomic region studied.

Labelling and hybridisation were performed following the protocols provided by Agilent (Agilent Oligonucleotide Array-Based CGH for Genomic DNA Analysis protocol v5.0) as described in [17]. Briefly, genomic DNA from a control and a patient in the same quantity were digested by restriction enzymes AluI and RsaI in separate tubes. The obtained fragments of DNA from each samples were amplified in presence of Cyanine 3-dUTP (control sample) and Cyanine 5-dUTP (patient sample). After a step of purification and quantitation of the incorporation of the Cyanines samples were combined and hybridized onto the microarray for 17 hours at 65°C.

The array was analysed with the Agilent scanner and the Feature Extraction software (v9.1). A graphical overview and analysis of the data were obtained using the DNA analytics software (v4.0.36). For identifying duplications and deletions the present inventors used the standard set-up of the ADM-2 statistical analysis provided by DNA analytics software (Agilent genomic Workbench 5.0) freely available at Agilent Technologies website.

According to this set-up and in the case of autosomal genes, heterozygous deletions are visualised with values of minus 1 and homozygous deletions as minus infinite (-4 in CGH analytics). The corresponding values for heterozygous and homozygous duplications are plus 0.5 and plus 1 respectively. At least 4 consecutive nonoverlapping probes reaching these values were needed for a positive call, together with absence of known SNPs in the region covered by the significant probes. All the 15 patients were done in duplicate on the array in order to provide robustness and validity to the results.

### Bioinformatics analysis of gene networks

Pathway analysis and literature mining was performed using Pathway Studio software from Ariadne Genomics Inc. Pathway Studio database contains millions of regulatory and interaction events from all Pubmed abstracts and more than 350,000 full-text articles extracted by MedScan natural language processing technology. The present inventors have used graph navigation tool in Pathway Studio called "Build pathway" to find literature evidence supporting functional association of measured genes with angiogenesis and other processes linked to blood vessel formation as well as immunity and neurodegeneration.

### Results

### CGH-ARRAY data

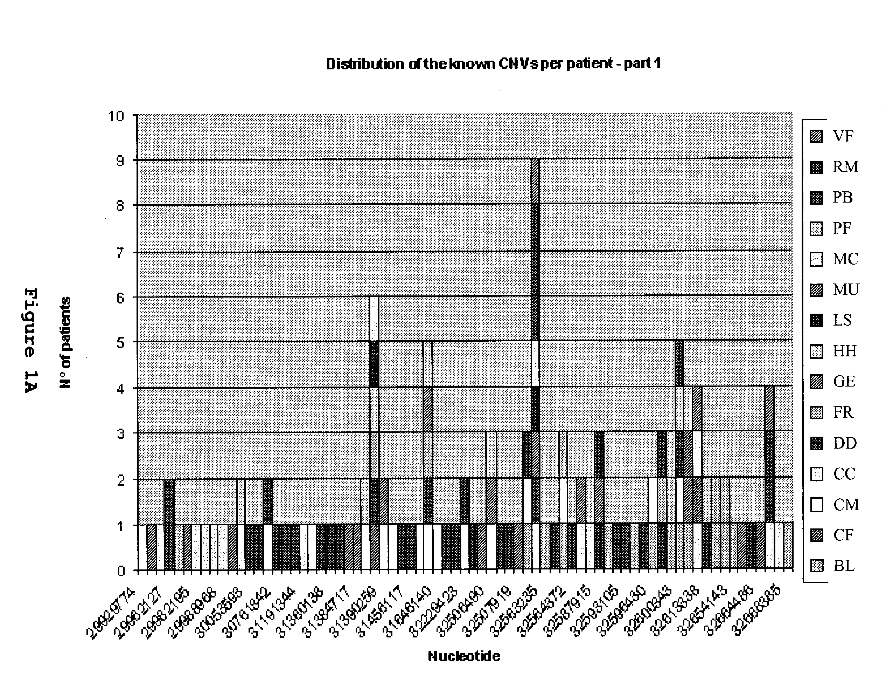

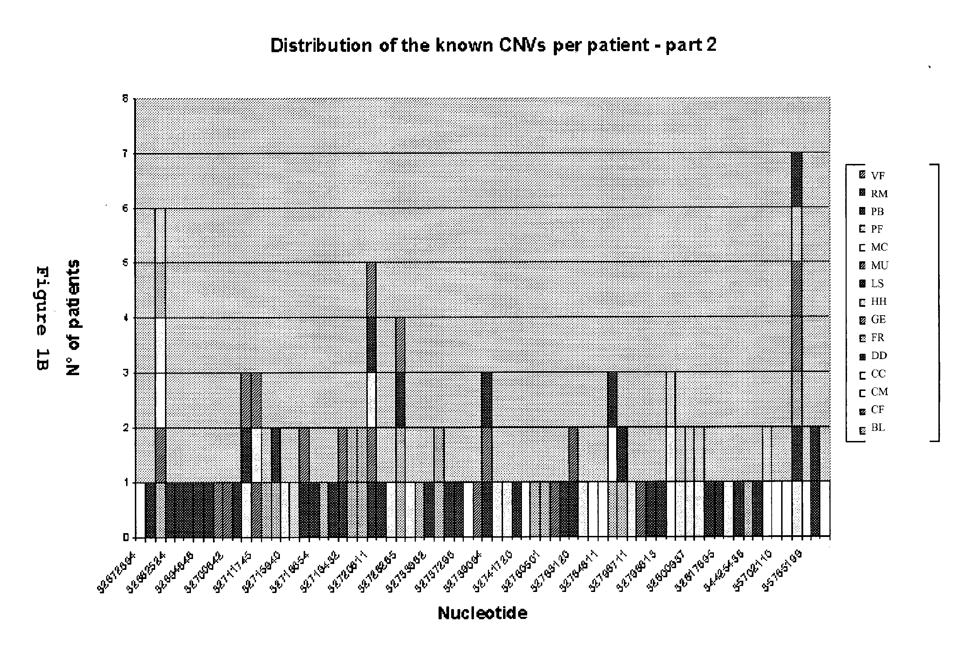

The present inventors identified 234 known polymorphic CNVs in the 15 patients by comparing with the CNVs database [18]. This finding confirmed that the HLA locus is highly polymorphic in terms of genomic unbalances as expected considering the known high density of SNPs. The distribution of the CNVs among patients both in terms of number and density of them is showed in Figure 1A and 1B.

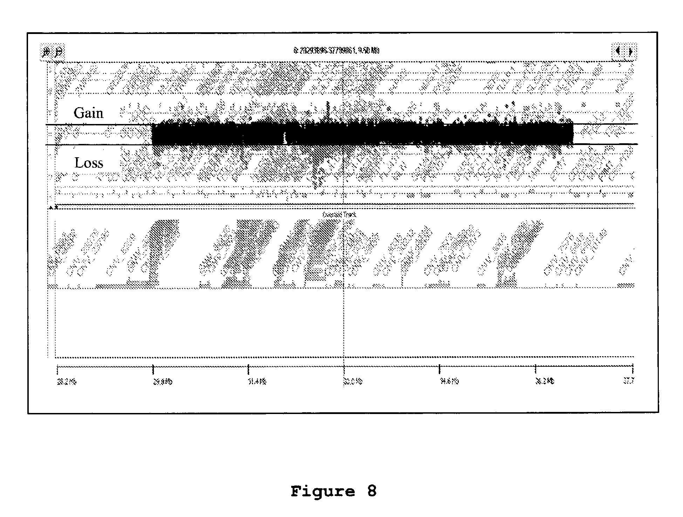

Table 3 reports all the CNVs identified in patients. CNVs in the last column of the table are indentified with same catalog number of the Database of Genomic Variants and can be searched either in UCSC Genome Browser and in the Database of Genomic Variants itself. Figure 8 reports on the CGH profile identified in patient PF, as example.

**Table 3.**

| **Patient Code** | **Chr.** | **Start** | **Stop** | **genes** | **gs_hg18_CNV_20080404** |
|---|---|---|---|---|---|
| BL | chr6 | 32536756 | 32766992 | | CNV_3603, CNV_4493, CNV_7567, CNV_32789, CNV_31279, CNV_31280, CNV_4767, CNV_23237, CNV_32790, CNV_32791, CNV_23801, CNV_1709, CNV_32792, CNV_23802, CNV_6759, CNV_6761, CNV_32793, CNV_32794, CNV_7568, CNV_23283, CNV_32795, CNV_6764, CNV_32796, CNV_7569 |
| BL | chr6 | 32564230 | 32565022 | | CNV_3603, CNV_4493, CNV_7567, CNV_31200, CNV_4767, CNV_23237, CNV_32790 |
| BL | chr6 | 32595206 | 32595421 | HLA-DRB5 | CNV_3603, CNV_4493, CNV_7567, CNV_31280, CNV_4767, CNV_23237 |
| BL | chr6 | 32596430 | 32597048 | HLA-DRB5 | CNV_3603, CNV_4493, CNV_7567, CNV_31280, CNV_4767, CNV_23237, CNV_32791 |
| BL | chr6 | 32600343 | 32605108 | HLA-DRB5 | CNV_3603, CNV_4493, CNV_7567, CNV_31280, CNV_4767, CNV_23237, CNV_32791 |
| BL | chr6 | 32630548 | 32633760 | HLA-DRB5 | CNV_3603, CNV_4493, CNV_7567, CNV_31280, CNV_4767, CNV_23237, CNV_32791 |
| BL | chr6 | 32644521 | 32654562 | HLA-DRB5 | CNV_3603, CNV_4493, CNV_7567, CNV_31280, CNV_4767, CNV_32792 |
| BL | chr6 | 32654143 | 32654521 | HLA-DRB5 | CNV_3603, CNV_4493, CNV_7567, CNV_31280, CNV_4767 |
| BL | chr6 | 32668385 | 32668850 | | CNV_3603, CNV_4493, CNV_7567, CNV_31280, CNV_4767 |
| BL | chr6 | 32680250 | 32680644 | | CNV_3603, CNV_4493, CNV_7567, CNV_31280, CNV_4767 |
| BL | chr6 | 32712472 | 32712943 | | CNV_3603, CNV_7567, CNV_31280, CNV_4767, CNV_7568 |
| BL | chr6 | 32720046 | 32720535 | HLA-DQA1 | CNV_3603, CNV_7567, CNV_31280, CNV_4767, CNV_7568, CNV_23283 |
| BL | chr6 | 32720570 | 32721540 | HLA-DQA1 | CNV_3603, CNV_7567, CNV_31280, CNV_4767, CNV_7568, CNV_23283 |
| BL | chr6 | 32728265 | 32728541 | | CNV_3603, CNV_7567, CNV_31280, CNV_4767, CNV_7568, CNV_23283 |
| BL | chr6 | 32728938 | 32731038 | | CNV_3603, CNV_7567, CNV_31280, CNV_4767, CNV_7568, CNV_23283 |
| BL | chr6 | 32734749 | 32739935 | HLA-DQB1 | CNV_3603, CNV_7567, CNV_31280, CNV_4767, CNV_7568 |
| BL | chr6 | 32786798 | 32787270 | | CNV_3603, CNV_4767, CNV_7569, CNV_31281 |
| BL | chr6 | 34425436 | 34426875 | NUDT3 | CNV_7572 |
| CF | chr6 | 31387332 | 31418253 | | CNV_3601, CNV_8131, CNV_5387, CNV_8508, CNV_5222, CNV_2624, CNV_31274, CNV_7563, CNV_7564, CNV_32780, CNV_1708, CNV_10145, CNV_10146, CNV_6758, CNV_23799 |
| CF | chr6 | 32680250 | 32680644 | | CNV_3603, CNV_4493, CNV_7567, CNV_31280, CNV_4767 |
| CM | chr6 | 29929774 | 29935498 | | CNV_8209, CNV_3599 |
| CM | chr6 | 30761724 | 30763025 | KIAA1949 | CNV_3600, CNV_31270 |
| CM | chr6 | 31311983 | 31314021 | | CNV_3601, CNV_8131, CNV_7561 |
| CM | chr6 | 31384822 | 31392869 | | CNV_3601, CNV_8131, CNV_5387, CNV_8508, CNV_5222, CNV_2624, CNV_31274, CNV_7563, CNV_7564, CNV_32780, CNV_1708, CNV_10145, CNV_10146, CNV_6758 |
| CM | chr6 | 31390259 | 31390381 | | CNV_3601, CNV_8131, CNV_5387, CNV_8508, CNV_5222, CNV_2624, CNV_31274, CNV_7564, CNV_32780, CNV_1708, CNV_10146 |
| CM | chr6 | 31392907 | 31418253 | | CNV_3601, CNV_8131, CNV_5387, CNV_8508, CNV_5222, CNV_2624, CNV_31274, CNV_7564, CNV_32780, CNV_10146, CNV_23799 |
| CM | chr6 | 31456523 | 31457088 | | CNV_3601, CNV_8131, CNV_7564 |
| CM | chr6 | 31457088 | 31457425 | | CNV_3601, CNV_8131, CNV_7564 |
| CM | chr6 | 31646140 | 31646463 | | CNV_2625 |
| CM | chr6 | 32229423 | 32230350 | PPT2 | CNV_3602 |
| CM | chr6 | 32506490 | 32506778 | | CNV_3603, CNV_4493 |
| CM | chr6 | 32536756 | 32531232 | | CNV_3603, CNV_4493, CNV_7567 |
| CM | chr6 | 32563017 | 32683706 | HLA-DRB5 | CNV_3603, CNV_4493, CNV_7567, CNV_31280, CNV_4767, CNV_23237, CNV_32790, CNV_32791, CNV_23801, CNV_1709, CNV_32792, CNV_23802, CNV_6759, CNV_6761 |
| CM | chr6 | 32564230 | 32564724 | | CNV_3603, CNV_4493, CNV_7567, CNV_31280, CNV_4767, CNV_23237, CNV_32790 |
| CM | chr6 | 32596430 | 32597048 | HLA-DRB5 | CNV_3603, CNV_4493, CNV_7567, CNV_31280, CNV_4767, CNV_23237, CNV_32791 |
| CM | chr6 | 32600343 | 32605108 | HLA-DRB5 | CNV_3603, CNV_4493, CNV_7567, CNV_31280, CNV_4767, CNV_23237, CNV_32791 |
| CM | chr6 | 32672694 | 32673267 | | CNV_3603, CNV_4493, CNV_7567, CNV_31280, CNV_4767 |
| CM | chr6 | 32680250 | 32680644 | | CNV_3603, CNV_4493, CNV_7567, CNV_31280, CNV_4767 |
| CM | chr6 | 32737867 | 32739304 | HLA-DQB1 | CNV_3603, CNV_7567, CNV_31280, CNV_4767, CNV_7568 |
| CM | chr6 | 32742604 | 32743626 | | CNV_3603, CNV_7567, CNV_31280, CNV_4767, CNV_7568 |
| CM | chr6 | 32767739 | 32772277 | | CNV_3603, CNV_4767, CNV_7569, CNV_32797 |
| CM | chr6 | 32784811 | 32785414 | | CNV_3603, CNV_4767, CNV_7569, CNV_31281 |
| CM | chr6 | 32786798 | 32786971 | | CNV_3603, CNV_4767, CNV_7569, CNV_31281 |
| CM | chr6 | 32799007 | 32799166 | | CNV_3603, CNV_32799 |
| CM | chr6 | 35586239 | 35586376 | TULP1 | CNV_3607 |
| CM | chr6 | 35702110 | 35704258 | FKBP5 | CNV_3607 |
| CM | chr6 | 35761904 | 35762281 | FKBP5 | CNV_3607, CNV_0286 |
| CM | chr6 | 35785199 | 35785703 | FKBP5 | CNV_3607, CNV_0286 |
| CM | chr6 | 35870799 | 35870982 | CLPS | CNV_3607, CNV_0286, CNV_31285, CNV_7575 |
| CC | chr6 | 31191344 | 31191466 | PSORS1C1 | CNV_3601, CNV_8131 |
| CC | chr6 | 32605201 | 32627687 | HLA-DRB5 | CNV_3603, CNV_4493, CNV_7567, CNV_31280, CNV_4767, CNV_23237, CNV_32791, CNV_23801 |
| CC | chr6 | 32680250 | 32680644 | | CNV_3603, CNV_4493, CNV_7567, CNV_31280, CNV_4767 |
| CC | chr6 | 32715940 | 32716099 | HLA-DQA1 | CNV_3603, CNV_7567, CNV_31280, CNV_4767, CNV_7568 |
| CC | chr6 | 32723584 | 32723758 | | CNV_3603, CNV_7567, CNV_31280, CNV_4767, CNV_7568, CNV_23283 |
| CC | chr6 | 32739728 | 32740502 | HLA-DQB1 | CNV_3603, CNV_7567, CNV_31280, CNV_4767, CNV_7568 |
| CC | chr6 | 32763291 | 32763593 | | CNV_3603, CNV 4767, CNV_7568 |
| CC | chr6 | 32796711 | 32797255 | | CNV_3603, CNV_32799 |
| CC | chr6 | 32799007 | 32799166 | | CNV_3603, CNV_32799 |
| CC | chr6 | 32800626 | 32800785 | | CNV_3603, CNV_32799, CNV_31282 |
| CC | chr6 | 32800937 | 32801062 | | CNV_3603, CNV_32799, CNV_31282 |
| CC | chr6 | 32801490 | 32801789 | | CNV_3603, CNV_32799, CNV_31282 |
| CC | chr6 | 32977765 | 32979689 | | CNV_0076, CNV_3604 |
| CC | chr6 | 35761943 | 35762129 | FKBP5 | CNV_3607, CNV_0286 |
| DD | chr6 | 29962127 | 30021448 | | CNV_8209, CNV_3599, CNV_7558, CNV_2622, CNV_9032, CNV_31269, CNV_32776, CNV_4669, CNV_6756, CNV_6763_{.} CNV_6760, CNV_6757, CNV_6765, CNV_9031, CNV_7956, CNV_1124, CNV_1126, CNV_23797, CNV_6486, CNV_32777 |
| DD | chr6 | 30053593 | 30053867 | HLA-29.1 | CNV_3599, CNV_2622, CNV_31269, CNV_4669 |
| DD | chr6 | 30063205 | 30063591 | HLA-29.1 | CNV_3599, CNV_2622, CNV_31269, CNV_4669 |
| DD | chr6 | 30761724 | 30763025 | KIAA1949 | CNV_3600, CNV_31270 |
| DD | chr6 | 30844693 | 30845056 | | CNV_3600 |
| DD | chr6 | 31157798 | 31159289 | | CNV_3601, CNV_31271 |
| DD | chr6 | 31345211 | 31345844 | | CNV_3601, CNV_8131, CNV_7561, CNV_31272, CNV_5387, CNV_8508, CNV_32779 |
| DD | chr6 | 31360138 | 31361802 | | CNV_3601, CMV_8131, CNV_5367, CNV_8508, CNV_5222, CNV_7562, CNV_2624, CNV_31279 |
| DD | chr6 | 31387332 | 31392906 | | CNV_3601, CNV_8131, CNV_5387, CNV_8508, CNV_5222, CNV_2624, CNV_31274, CNV_7563, CNV_7564, CNV_32780, CNV_1708, CNV_10145, CNV_10146, CNV_6758 |
| DD | chr6 | 31393365 | 31418253 | | CNV_3601, CNV_8131, CNV_5387, CNV_8508, CNV_5222, CNV_2624, CNV_31274, CNV_7564, CNV_32780, CNV_10146, CNV_23799 |
| DD | chr6 | 31457088 | 31457425 | | CNV_3601, CNV_8131, CNV_7564 |
| DD | chr6 | 32229423 | 32230350 | PPT2 | CNV_3602 |
| DD | chr6 | 32507789 | 32508030 | | CNV_3603, CNV_4493 |
| DD | chr6 | 32563017 | 32733981 | HLA-DRB5 | CNV_3603, CNV_4493, CNV_7567, CNV_31280, CNV_4767, CNV_23237, CNV_32790, CNV_32791, CNV_23801, CNV_1709, CNV_32792, CNV_23802, CNV_6759, CNV_6761, CNV_32793, CNV_32794, CNV_7568, CNV_23283 |
| DD | chr6 | 32564372 | 32564724 | | CNV_3603, CNV_4493, CNV_7567, CNV_31280, CNV_4767, CNV_23237, CNV_32790 |
| DD | chr6 | 32587915 | 32592942 | | CNV_3603, CNV_4493, CNV_7567, CNV_31280, CNV_4767, CNV_23237, CNV_32790 |
| DD | chr6 | 32591668 | 32592671 | | CNV_3603, CNV_4493, CNV_7567, CNV_31280, CNV_4767, CNV_23237, CNV_32790 |
| DD | chr6 | 32596475 | 32597048 | HLA-DRB5 | CNV_3603, CNV_4493, CNV_7567, CNV_31280, CNV_4767, CNV_23237, CNV_32791 |
| DD | chr6 | 32600343 | 32605108 | HLA-DRB5 | CNV_3603, CNV_4493, CNV_7567, CNV_31280, CNV_4767, CNV_23237, CNV_32791 |
| DD | chr6 | 32658647 | 32659246 | HLA-DRB5 | CNV_3603, CNV_4493, CNV_7567, CNV_31280, CNV_4767 |
| DD | chr6 | 32689971 | 32716235 | HLA-DQA1 | CNV_3603, CNV_4493, CNV_7567, CNV_31290, CNV_4767, CNV_32793, CNV_32794, CNV_7568 |
| DD | chr6 | 32699139 | 32699290 | | CNV_3603, CNV_7567, CNV_31280, CNV_4767, CNV_32793 |
| DD | chr6 | 32712472 | 32712943 | | CNV_3603, CNV_7567, CNV_31280, CNV_4767, CNV_7568 |
| DD | chr6 | 32718420 | 32731038 | HLA-DQA1 | CNV_3603, CNV_7567, CNV_31280, CNV_4767, CNV_7568, CNV 23283 |
| DD | chr6 | 32718654 | 32718952 | HLA-DQA1 | CNV_3603, CNV_7567, CNV_31280, CNV_4767, CNV_7568, CNV_23283 |
| DD | chr6 | 32719432 | 32720046 | HLA-DQA1 | CNV_3603, CNV_7567, CNV_31280, CNV_4767, CNV 7568, CNV 23283 |
| DD | chr6 | 32720611 | 32721540 | HLA-DQA1 | CNV_3603, CNV_7567, CNV_31280, CNV_4767, CNV_7568, CNV 23283 |
| DD | chr6 | 32723425 | 32728264 | | CNV_3603, CNV_7567, CNV_31280, CNV_4767, CNV_7568, CNV_23283 |
| DD | chr6 | 32739094 | 32739774 | HLA-DQB1 | CNV_3603, CNV_7567, CNV_31280, CNV_4767, CNV_7568 |
| DD | chr6 | 32761602 | 32762261 | | CNV_3603, CNV_31280, CNV_4767, CNV_7568 |
| DD | chr6 | 32763120 | 32763367 | | CNV_3603, CNV_4767, CNV_7568 |
| DD | chr6 | 32786798 | 32790418 | | CNV_3603, CNV_4767, CNV_7569, CNV_31281 |
| DD | chr6 | 32798813 | 32800626 | | CNV_3603, CNV_32799, CNV_31282 |
| DD | chr6 | 32810657 | 32810878 | | CNV_3603 |
| DD | chr6 | 32817895 | 32818011 | HLA-DQA2 | CNV_7570 |
| DD | chr6 | 33069396 | 33075621 | | CNV_0076, CNV_3604 |
| DD | chr6 | 34773702 | 34779441 | | CNV_3605 |
| DD | chr6 | 35761943 | 35762075 | FKBP5 | CNV_3607, CNV_0286 |
| DD | chr6 | 35862999 | 35868527 | C6orf127 | CNV_3607, CNV_0286, CNV_31285, CNV 7515 |
| FR | chr6 | 29962649 | 30017382 | | CNV_8209, CNV_3599, CNV_7558; CNV_2622, CNV_9032, CNV_31269, CNV_32776, CNV_4669, CNV_6756, CNV_6763, CNV_6760, CNV_6757, CNV_6765, CNV_9031, CNV_7956, CNV_1124, CNV_1126, CNV_23797, CNV_6486 |
| FR | chr6 | 31387332 | 31418253 | | CNV_3601, CNV_8131, CNV_5387, CNV_8508, CNV_5222, CNV_2624, CNV_31274, CNV_7563, CNV_7564, CNV_32780, CNV_1708, CNV_10145, CNV_10146, CNV_6758, CNV_23799 |
| FR | chr6 | 31457088 | 31457425 | | CNV_3601, CNV_8131, CNV_7564 |
| FR | chr6 | 32563235 | 32740502 | HLA-DRBS | CNV_3603, CNV_4493, CNV_7567, CNV_31280, CNV_4767, CNV_23237, CNV_32790, CNV_32791, CNV_23801, CNV_1709, CNV_32792, CNV_23802, CNV_6759, CNV_6761, CNV_32793, CNV_32794, CNV_7568, CNV_23283 |
| FR | chr6 | 32569230 | 32565022 | | CNV_36C3, CNV_4493, CNV_7567, CNV_31200, CNV_4767, CNV_23237, CNV_32790 |
| FR | chr6 | 32590368 | 32592554 | | CNV_3603, CNV_4493, CNV_7567, CNV_31280, CNV_4767, CNV_23237, CNV_32790 |
| FR | chr6 | 32596475 | 32597048 | HLA-DRB5 | CNV_3603, CNV_4493, CNV_7567, CNV_31280, CNV_4767, CNV_23237, CNV_32791 |
| FR | chr6 | 32599429 | 32600342 | HLA-DRB5 | CNV_3603, CNV_4493, CNV_7567, CNV_31280, CNV_4767, CNV_23237, CNV_32791 |
| FR | chr6 | 32600917 | 32605108 | HLA-DRB5 | CNV_3603, CNV_4493, CNV_7567, CNV_31280, CNV_4767, CNV_23237, CNV_32791 |
| FR | chr6 | 32630548 | 32631353 | HLA-DRB5 | CNV_3603, CNV_4493, CNV_7567, CNV_31280, CNV_4767, CNV_23237, CNV 32791 |
| FR | chr6 | 32644521 | 32650183 | HLA-DRB5 | CNV_3603, CNV_4493, CNV_7567, CNV_31280, CNV_4767, CNV 32792 |
| FR | chr6 | 32680250 | 32680699 | | CNV_3603, CNV_4493, CNV_7567, CNV_31280, CNV_4767 |
| FR | chr6 | 32712300 | 32712943 | | CNV_3603, CNV_7567, CNV_31280, CNV_4767, CNV_7568 |
| FR | chr6 | 32719305 | 32719932 | HLA-DQA1 | CNV_3603, CNV_7567, CNV_31280, CNV_4767, CNV_7568, CNV_23283 |
| FR | chr6 | 32720046 | 32720535 | HLA-DQA1 | CNV_3603, CNV_7567, CNV_31280, CNV_4767, CNV_7568; CNV_23283 |
| FR | chr6 | 32720570 | 32721540 | HLA-DQA1 | CNV_3603, CNV_7567, CNV_31280, CMV_4767, CNV_7568, CNV_23283 |
| FR | chr6 | 32728265 | 32728737 | | CNV_3603, CNV_7567, CNV_31280, CNV_4767, CNV_7568, CNV_23283 |
| FR | chr6 | 32734749 | 32734935 | | CNV_3603, CNV_7567, CNV_31280, CNV_4767, CNV_7568 |
| FR | chr6 | 32743627 | 32744515 | | CNV_3603, CNV_7567, CNV_31280, CNV_4767, CNV_7568 |
| FR | chr6 | 32760501 | 32761273 | | CNV_3603, CNV_7567, CNV_31280, CNV_4767, CNV_7568 |
| FR | chr6 | 32786937 | 32787270 | | CNV_3603, CNV_4767, CNV_7569, CNV_31281 |
| FR | chr6 | 35761943 | 35762281 | FKBP5 | CNV_3607, CNV_0286 |
| GE | chr6 | 29940096 | 30017382 | | CNV_8209, CNV_3599, CNV_7558, CNV_2622, CNV_9032, CNV_31269, CNV_23095, CNV_22632, CNV_23796, CNV_32776, CNV_4669, CNV_6756, CNV_6763, CNV_6760, CNV_6757, CNV_6765, CNV_9031, CNV_7956, CNV_1124, CNV_1126, CNV_23797. CNV_6486 |
| GE | chr6 | 29981807 | 29982868 | | CNV_8209, CNV_3599, CNV_7558, CNV_2622, CNV_9032, CNV_31269, CNV 32776, CNV 4669, CNV 9031, CNV_7956 |
| GE | chr6 | 31367445 | 31367910 | | CNV_3601, CNV_8131, CNV_5387, CNV_8508, CNV_5222, CNV_2624, CNV_31274 |
| GE | chr6 | 31384717 | 31418029 | | CNV_3601, CNV_8131, CNV_5387, CNV_8508, CNV_5222, CNV_2624, CNV_31274, CNV_7563, CNV_7564, CNV_32780, CNV_1708, CNV_10145, CNV_10146, CNV_6758, CNV_23799 |
| GE | chr6 | 31390259 | 31390381 | | CNV_3601, CNV_8131, CNV_5387, CNV_8508, CNV_5222, CNV_2624, CNV_31274, CNV_7564, CNV_32780, CNV_1708, CNV_10146 |
| GE | chr6 | 31457088 | 31457425 | | CNV 3601, CNV_8131, CNV_7564 |
| GE | chr6 | 32477682 32506490 | 32477991 32506778 | BTNL2 | CNV_7566 CNV_3603, CNV_4493 |
| GE | chr6 | 32507919 | 32508030 | | CNV_3603, CNV_4493 |
| GE | chr6 | 32563017 | 32744157 | HLA-DRB5 | CNV_3603, CNV_4493, CNV_7567, CNV_31280, CNV_4767, CNV_23237, CNV_32790, CNV_32791, CNV_23801, CNV_1709, CNV_32792, CNV_23802, CNV_6759, CNV_6761, CNV_32793, CNV_32794, CNV_7568, CNV_23283 |
| GE | chr6 | 32587915 | 32592942 | | CNV_3603, CNV_4493, CNV_7567, CNV_31280, CNV_4767, CNV_23237, CNV_32790 |
| GE | chr6 | 32600917 | 32605108 | HLA-DRB5 | CNV_3603, CNV_4493, CNV_7567, CNV_31280, CNV_4767, CNV_23237, CNV_32791 |
| GE | chr6 | 32658039 | 32659542 | HLA-DRB5 | CNV 3603, CNV 4493, CNV 7567, CNV 31280, CNV 4767 |
| GE | chr6 | 32699503 | 32701186 | | CNV 3603, CNV_7567, CNV_31280, CNV 4767, CNV 32793 |
| GE | chr6 | 32718420 | 32723425 | HLA-DQA1 | CNV_3603, CNV_7567, CNV_31280, CNV_4767, CNV_7568, CNV_23283 |
| GE | chr6 | 32719432 | 32720268 | HLA-DQA1 | CNV_3603, CNV_7567, CNV_31280, CNV_4767, CHV_7568, CNV_23283 |
| GE | chr6 | 32720611 | 32721540 | HLA-DQA1 | CNV_3603, CNV_7567, CNV_31280, CNV_4767, CNV_7568, CNV_23283 |
| GE | chr6 | 32738284 | 32738429 | HLA-DQB1 | CNV_3603, CNV_7567, CNV_31280, CNV_4767, CNV_7568 |
| GE | chr6 | 32739094 | 32739452 | HLA-DQB1 | CNV_3603, CNV_7567, CNV_31280, CNV_4767, CNV_7568 |
| GE | chr6 | 35761943 | 35762281 | FKBP5 | CNV_3607, CNV_0286 |
| HH | chr6 | 31387332 | 31418253 | | CNV_3601, CNV_8131, CNV_5387, CNV_8508, CNV_5222, CNV_2624, CNV_31274, CNV_7563, CNV_7564, CNV_32780, CNV_1708, CNV_10145, CNV_10146, CNV_6758, CNV_23799 |
| LS | chr6 | 31387332 | 31392974 | | CNV_3601, CNV_8131, CNV_5387, CNV_8508, CNV_5222, CNV_2624, CNV_31274, CNV_7563, CNV_7564, CNV_32780, CNV_1708, CNV_10145, CNV_10146, CNV_6758 |
| LS | chr6 | 32563017 | 32563369 | | CNV_3603, CNV_4493, CNV_7567, CNV_31280, CNV_4767, CNV_23237 |
| MU | chr6 | 29995047 | 29998209 | | CNV_8209, CNV_3599, CNV_7558, CNV_2622, CNV_9032, CNV_31269, CNV_32776, CNV_4669, CNV_9031, CNV_7956, CNV_1124, CNV_1126 |
| MU | chr6 | 32600917 | 32604580 | HLA-DRB5 | CHV_3603, CNV_9493, CNV_7567, CNV_31280, CNV_4767, CNV_23237, CNV_32791 |
| MU | chr6 | 32605201 | 32628505 | HLA-DRB5 | CNV_3603, CNV_4493, CNV_7567, CNV_31280, CNV_4767, CNV_23237, CNV_32791, CNV_23801 |
| MU | chr6 | 32664486 | 32667076 | HLA-DRB5 | CNV 3603, CNV 4493, CNV_7567, CNV_31280, CNV_4767 |
| MU | chr6 | 32700842 | 32700989 | | CNV 3603, CNV 7567, CNV_31280, CNV 4767, CNV 32793 |
| MU | chr6 | 32711745 | 32712127 | | CNV_3603, CNV_7567, CNV_31280, CNV_4767, CNV_32794, CNV_7568 |
| MU | chr6 | 32760784 | 32761389 | | CNV_3603, CNV_7567, CNV_31280, CNV_4767, CNV_7568 |
| MU | chr6 | 32763120 | 32763593 | | CNV 3603, CNV_4767, CNV_7568 |
| MU | chr6 | 32798352 | 32802221 | | CNV 3603, CNV_32799, CNV_31282 |
| MU | chr6 | 35761943 | 35762281 | FKBP5 | CNV_3607, CNV_0286 |
| MC | chr6 | 29950578 | 30017155 | | CNV_8209, CNV_3599; CNV_7558, CNV_2622, CNV_9032, CNV_31269, CNV_23095, CNV_22632, CNV_23796, CNV_32776, CNV_4669, CNV_6756, CNV_6763, CNV_6760, CNV_6757, CNV_6765, CNV_9031, CNV_7956, CNV_1124, CNV_1126, CNV_23797, CNV_6486 |
| MC | chr6 | 29982195 | 29982333 | | CNV_8209, CNV_3599, CNV_7558, CNV_2622, CNV_9032, CNV_31269, CNV_32776, CNV_4669, CNV_9031, CNV_7956 |
| MC | chr6 | 29985562 | 30001164 | | CNV_8209, CNV_3599, CNV_7558, CNV_2622, CNV_9032, CNV_31269, CNV_32776, CNV_4669, CNV_9031, CNV_7956, CNV_1124, CNV_1126 |
| MC | chr6 | 29985909 | 29986570 | | CNV_8209, CNV_3599, CNV_7558, CNV_2622, CNV_9032, CNV_31269, CNV_32776, CNV_4669, CNV_9031, CNV_7956 |
| MC | chr6 | 29988968 | 29994086 | | CNV_8209, CNV_3599, CNV_7558, CNV_2622, CNV-9032, CNV_31269, CNV_32776, CNV_4669, CNV_9031, CNV_7956, CNV_1124, CNV_1126 |
| MC | chr6 | 30006771 | 30010434 | | CNV_8209, CNV_3599, CNV_7558, CNV_2622, CNV_9032, CNV_31269, CNV_32776, CNV_4669, CNV_9031, CNV_7956, CNV_23797, CNV_6486 |
| MC | chr6 | 31387332 | 31418029 | | CNV_3601, CNV_8131, CNV_5387, CNV_8508, CNV_5222, CNV_2624, CNV_31274, CNV_7563, CNV_7564, CNV_32780, CNV_1708, CNV_10145, CNV_10146, CNV_6758, CNV_23799 |
| MC | chr6 | 32563017 | 32567703 | | CNV_3603, CNV_4493, CNV_7567, CNV_31280, CNV_4767, CNV_23237, CNV_32790 |
| MC | chr6 | 32564548 | 32565176 | | CNV_3603, CNV_4493, CNV_7567, CNV_31280, CNV_4767, CNV_23237, CNV_32790 |
| MC | chr6 | 32587803 | 32588104 | | CNV_3603, CNV_4493, CNV_7567, CNV_31280, CNV_4767, CNV_23237, CNV_32790 |
| MC | chr6 | 32605201 | 32605946 | HLA-DRB5 | CNV_3603, CNV_4493, CNV_7567, CNV_31280, CNV_4767, CNV_23237, CNV_32791 |
| MC | chr6 | 32667923 | 32668136 | | CNV_3603, CNV_4493, CNV_7567, CNV_31280, CNV_4767 |
| MC | chr6 | 32668136 | 32668384 | | CNV_3603, CNV_4493, CNV_7567, CNV_31280, CNV_4767 |
| MC | chr6 | 32703944 | 32707105 | | CNV_3603, CNV_7567, CNV_31280, CNV_4767, CNV_32793 |
| MC | chr6 | 32711745 | 32712681 | | CNV_3603, CNV_7567, CNV_31280, CNV_4767, CNV_32794, CNV_7568 |
| MC | chr6 | 32720611 | 32721540 | HLA-DQA1 | CNV_3603, CNV_7567, CNV_31280, CNV_4767, CNV_7568, CNV_23283 |
| MC | chr6 | 32728386 | 32728737 | | CNV_3603, CNV_7567, CNV_31280, CNV_4767, CNV_7568, CNV_23283 |
| MC | chr6 | 32739606 | 32740000 | HLA-DQB1 | CNV 3603, CNV 7567, CNV 31280, CNV 4767, CNV 7568 |
| PF | chr6 | 30006771 | 30010434 | | CNV_8209, CNV_3599, CNV_7558, CNV_2622, CNV_9032, CNV_31269, CNV_32776, CNV_4669, CNV_9031, CNV_7956, CNV_23797, CNV_6486 |
| PF | chr6 | 31384822 | 31386601 | | CNV_3601, CNV_8131, CNV_5387, CNV_8508, CNV_5222, CNV_2624, CNV_31274, CNV_7563, CNV_7564, CNV_32780, CNV_1708, CNV_10145 |
| PF | chr6 | 31457088 32506490 | 31457425 32506778 | | CNV_3601, CNV_8131, CNV_7564 CNV_3603, CNV_4493 |
| PF | chr6 | 32600343 | 32605108 | HLA-DRB5 | CNV_3603, CNV_4493, CNV_7567, CNV_31280, CNV_4767, CNV_23237, CNV_32791 |
| PF | chr6 | 32680250 | 32680644 | | CNV_3603, CNV_4493, CNV_7567, CNV_31280, CNV_4767 |
| PF | chr6 | 32717273 | 32717335 | HLA-DQA1 | CNV_3603, CNV_7567, CNV_31280, CNV_4767, CNV_7568 |
| PF | chr6 | 32799007 | 32799166 | | CNV_3603, CNV_32799 |
| PF | chr6 | 32800937 | 32801062 | | CNV_3603, CNV_32799, CNV_31282 |
| PF | chr6 | 32801490 | 32801789 | | CNV_3603, CNV_32799, CNV_31282 |
| PF | chr6 | 35586239 | 35586376 | TULP1 | CNV_3607 |
| PF | chr6 | 35761943 | 35762281 | FKBP5 | CNV_3607, CNV_0286 |
| PB | chr6 | 31456117 | 31456849 | | CNV_3601, CNV_8131, CNV 7564 |
| PB | chr6 | 32563017 | 32605362 | HLA-DRB5 | CNV_3603, CNV_4493, CNV_7567, CNV_31280, CNV_4767, CNV_23237, CNV_32790, CNV_32791 |
| PB | chr6 | 32593105 | 32598770 | HLA-DRB5 | CNV_3603, CNV_4493, CNV_7567, CNV_31280, CNV_4767, CNV_23237, CNV_32791 |
| PB | chr6 | 32596367 | 32597048 | HLA-DRB5 | CNV_3603. CNV_4493, CNV_7567, CNV_31280, CNV_4767, CNV_23237, CNV_32791 |
| PB | chr6 | 32667923 | 32668384 | | CNV_3603, CNV_4493, CNV_7567, CNV_31280, CNV_4767 |
| PB | chr6 | 32694846 | 32696612 | | CNV_3603, CNV_7567, CNV_31280, CNV_4767 |
| PB | chr6 | 32737298 | 32739452 | HLA-DQB1 | CNV_3603, CNV_7567, CNV_31280, CNV_4767, CNV_7568 |
| PB | chr6 | 32741720 | 32741876 | HLA-DQB1 | CNV_3603, CNV_7567, CNV_31280, CNV_4767, CNV_7568 |
| RM | chr6 | 29962127 | 30017382 | | CNV_8209, CNV_3599, CNV_7558, CNV_2622, CNV_9032, CNV_31269, CNV_32776, CNV_4669, CNV_6756, CNV_6763, CNV_6760, CNV_6757, CNV_6765, CNV_9031, CNV_7956, CNV_1124, CNV_1126, CNV_23797, CNV_6486 |
| RM | chr6 | 30761842 | 30763025 | KIAA1949 | CNV_3600, CNV_31270 |
| RM | chr6 | 31361260 | 31361802 | | CNV_3601, CNV_8131, CNV_5387, CNV_8508, CNV_5222, CNV_7562, CNV_2624, CNV_31274 |
| RM | chr6 | 32226417 | 32226779 | PRRT1 | CNV_3602 |
| RM | chr6 | 32229121 | 32230350 | PPT2 | CNV_3602 |
| RM | chr6 | 32260717 | 32261345 | PBX2 | CNV_3602 |
| RM | chr6 | 32507870 | 32508030 | | CNV 3603, CNV 4493 |
| RM | chr6 | 32536756 | 32537232 | | CNV_3603, CNV 4493, CNV 7567 . |
| RM | chr6 | 32563017 | 32763367 | HLA-DRB5 | CNV_3603, CNV_4493, CNV_7567, CNV_31280, CNV_4767, CNV_23237, CNV_32790, CNV_32791, CNV_23801, CNV_1709, CNV_32792, CNV₋23802, CNV_6759, CNV_6761, CNV_32793, CNV_32794, CNV_7568, CNV_23283, CNV_32795, CNV_6764 |
| RM | chr6 | 32563017 | 32592942 | | CNV_3603, CNV_4493, CNV_7567, CNV_31280, CNV_4767, CNV_23237, CNV_32790 |
| RM | chr6 | 32564010 | 32564512 | | CNV_3603, CNV_4493, CNV_7567, CNV_31280, CNV_4767, CNV_23237, CNV_32790 |
| RM | chr6 | 32587915 | 32588033 | | CNV_3603, CNV_4493, CNV_7567, CNV_31280, CNV_4767, CNV_23237, CNV_32790 |
| RM | chr6 | 32596475 | 32597048 | HLA-DRB5 | CNV_3603, CNV_4493, CNV_7567, CNV_31280, CNV_4767, CNV_23237, CNV_32791 |
| RM | chr6 | 32600343 | 32605946 | HLA-DRB5 | CNV_3603, CNV_4493, CNV_7567, CNV_31280, CNV_4767, CNV_23237, CNV_32791 |
| RM | chr6 | 32613338 | 32627232 | HLA-DRB5 | CNV_3603, CNV_4493, CNV_7567, CNV_31280, CNV_4767, CNV_23237, CNV_32791 |
| RM | chr6 | 32667923 | 32668136 | | CNV_3603, CNV_4493, CNV_7567, CNV_31280, CNV_4767 |
| RM | chr6 | 32679817 | 32680250 | | CNV_3603, CNV_4493, CNV_7567, CNV_31280, CNV_4767 |
| RM | chr6 | 32682524 | 32683273 | | CNV_3603, CNV_4493, CNV_7567, CNV_31280, CNV_4767, CNV_6759, CNV_6761 |
| RM | chr6 | 32693553 | 32703913 | 32703913 | CNV_3603, CNV_4493, CNV_7567, CNV_31280, CNV_4767, CNV_32793 |
| RM | chr6 | 32702660 | 32702818 | | CNV_3603, CNV_7567, CNV_31280, CNV_4767, CNV_32793 |
| RM | chr6 | 32703944 | 32707105 | | CNV_3603, CNV_7567, CNV_31280, CNV_4767, CNV_32793 |
| RM | chr6 | 32719403 | 32719774 | HLA-DQA1 | CNV_3603, CNV_7567, CNV_31280, CNV_4767, CNV_7568, CNV_23283 |
| RM | chr6 | 32720611 | 32721540 | HLA-DQA1 | CNV_3603, CNV_7567, CNV_31280, CNV_4767, CNV_7568, CNV_23283 |
| RM | chr6 | 32728265 | 32731038 | | CNV_3603, CNV_7567, CNV_31280, CNV_4767, CNV_7568, CNV_23283 |
| RM | chr6 | 32733982 | 32759283 | HLA-DQB1 | CNV_3603, CNV_7567, CNV_31280, CNV_4767, CNV_7568, CNV_32795, CNV_6764 |
| RM | chr6 | 32735643 | 32735978 | HLA-DQB1 | CNV_3603, CNV_7567, CNV_31280, CNV_4767, CNV_7568 |
| RM | chr6 | 32739094 | 32739452 | HLA-DQB1 | CNV_3603, CNV_7567, CNV_31280, CNV_4767, CNV_7568 |
| RM | chr6 | 32786937 | 32790418 | | CNV_3603, CNV_4767, CNV_7569, CNV_31281 |
| RM | chr6 | 32798570 | 32801825 | | CNV_3603, CNV_32799, CNV_31282 |
| RM | chr6 | 35761943 | 35762281 | FKBP5 | CNV_3607, CNV_0286 |
| RM | chr6 | 35862999 | 35868527 | C6orf127 | CNV_3607, CNV_0286, CNV_31285, CNV_7575 |
| VF | chr6 | 32563017 | 32588104 | | CNV_3603, CNV_4493, CNV_7567, CNV_31280, CNV_4767, CNV_23237, CNV_32790 |
| VF | chr6 | 32564548 | 32565176 | | CNV_3603, CNV_4493, CNV_7567, CNV_31280, CNV_4767, CNV_23237, CNV_32790 |
| VF | chr6 | 32605201 | 32605362 | HLA-DRB5 | CNV_3603, CNV_4493, CNV_7567, CNV_31280, CNV_4767, CNV_23237, CNV_32791 |
| VF | chr6 | 32667923 | 32668136 | | CNV_3603, CNV_4493, CNV_7567, CNV_31280, CNV_4767 |
| VF | chr6 | 32703944 | 32707105 | | CNV_3603, CNV_7567, CNV_31280, CNV_4767, CNV_32793 |
| VF | chr6 | 32711745 | 32712943 | | CNV_3603, CNV_7567, CNV_31280, CNV_4767, CNV_32794, CNV_7568 |
| VF | chr6 | 32720611 | 32721540 | HLA-DQA1 | CNV_3603, CNV_7567, CNV_31280, CNV_4767, CNV_7568, CNV_23283 |
| VF | chr6 | 32728265 | 32728737 | | CNV_3603, CNV_7567, CNV_31280, CNV_4767, CNV_7568, CNV_23283 |

### Genotype-phenotype correlation: statistical analysis

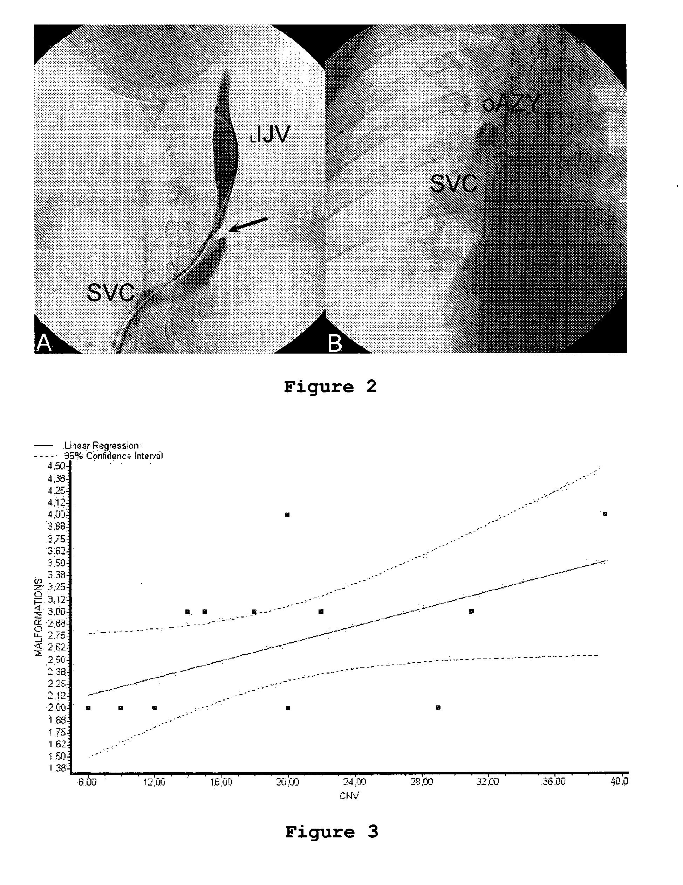

The present inventors correlated the number of the CNVs, with the clinical parameters of the patients (Figure 2, top panel, Table 1). For the oversized number of CNVs significantly affecting X and Y Intercept, Slope and Run Test the present inventors excluded from linear regression analysis the patient coded as MC (52 CNVs vs median (IR) 23 (13) of our patients population, Table 1).

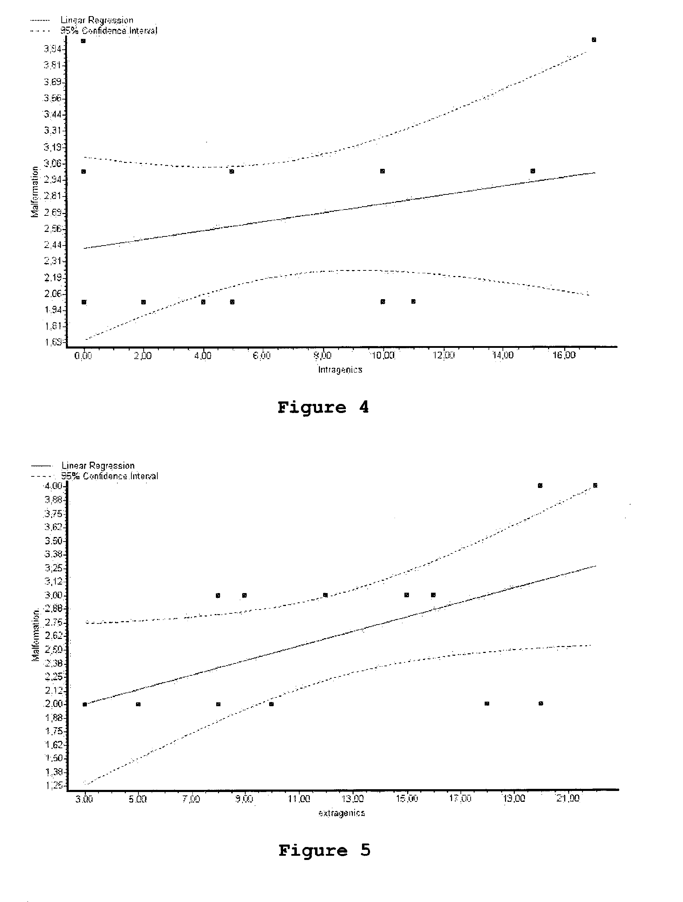

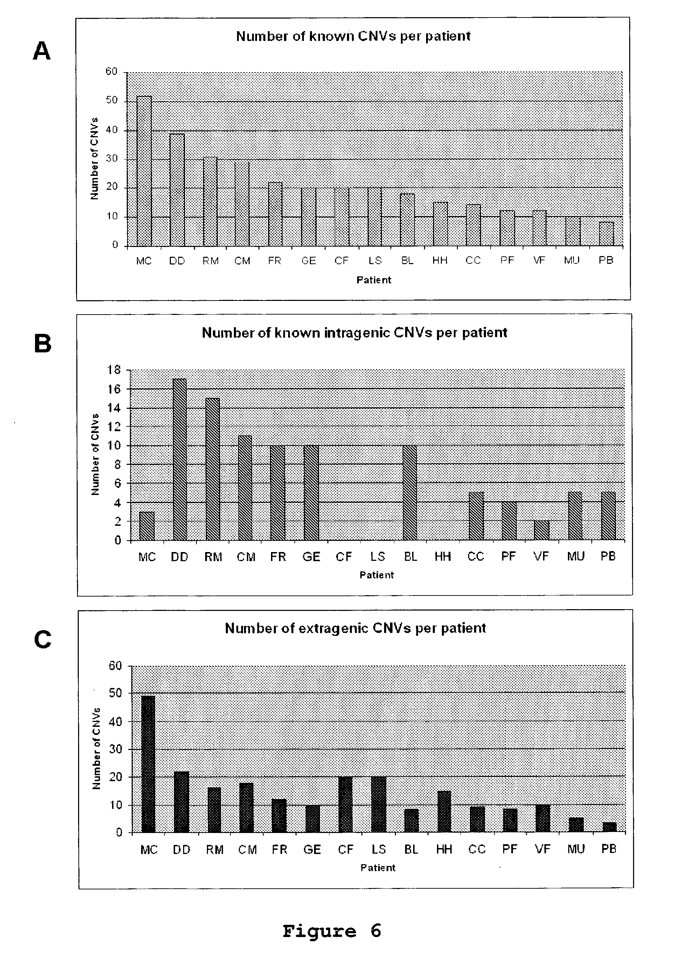

Linear regression analysis demonstrated that the overall number of CNVs is correlated with the number of extracranial VM demonstrated by venography in the extracranial segments of the cerebrospinal veins, with a trend toward significance (r=0.52, r2=0.27, p=0.0545) (Figure 3 and Figure 6 panel A). By splitting the analysis correlating either the extragenic CNVs (Figure 5 and Figure 6 panel C) or the intragenic CNVs (Figure 4 and Figure 6 panel B) with the extracranial VM the present inventors found a robust and significant correlation with the former (r=0.53, r2=0.28, p<0.05).

### Pathways and genes network functional bioinformatics analysis

211 genes are contained within the region the present inventors covered by the present CGH array.

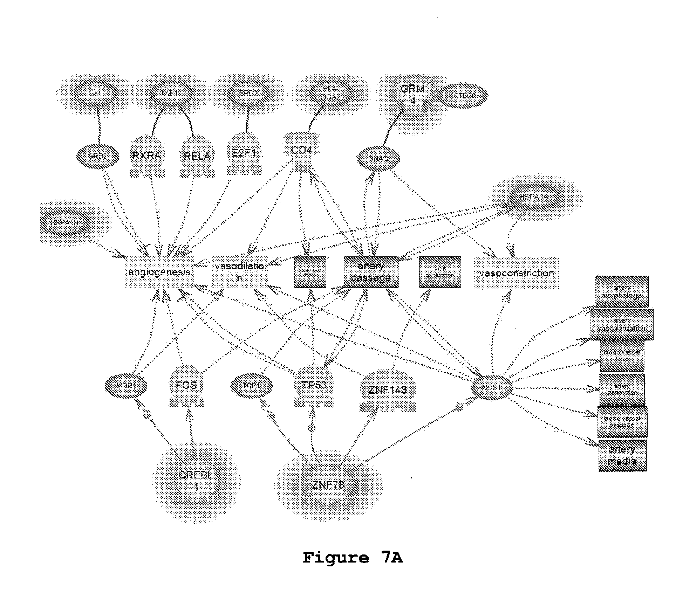

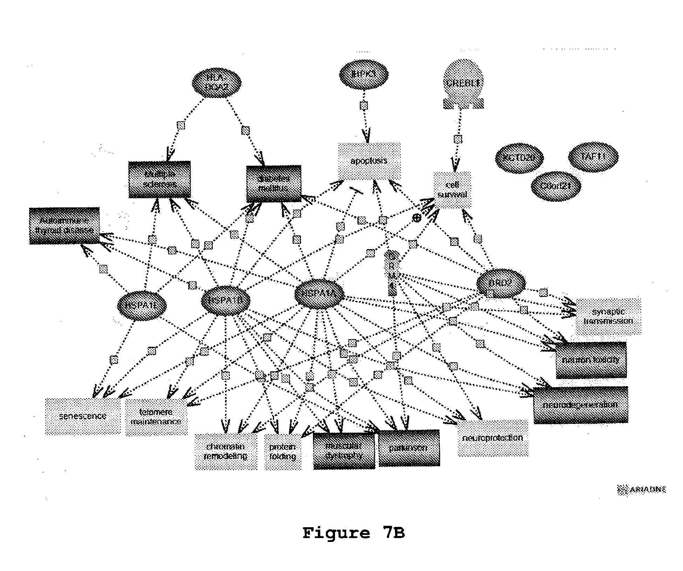

Since the phenotype in focus is characterised by multiple sclerosis and venous malformation, the present inventors applied a bioinformatics tool to select genes known to be involved in angiogenesis and venous development as well as linked to multiple sclerosis, immunity and neurodegeneration. Interestingly some genes are linked to these processes. HSPA1L and HSPA1A are linked to MS and diabetes and other immunity disorders, and regulatory functions as chromatin remodelling, neuroprotection, protein folding, and regenerative-degenerative tools as neurodegeneration, neuron toxicity, cell survival, neuroprotection, synaptic transmission or even aging factors (senescence and telomere maintenance). Also the gene GRM4 seems to interact with many proteins linked to MS (Figure 7A)
Focusing on specific functional pathways, as angiogenesis, obviously considering the plus phenotype of the present patients (venous malformation) the present inventors obtained a more selective puzzle of interactions (Figure 7B). GRB2 and HSPA1A and B genes directly act on angiogenesis, TAF11 is known to be involved in artery passage and E2F1 transcription factor is known to be an angiogenesis positive inducer in hepatitis and cancer. Interesting HLA-DQA2 may also be implicated in angiogenesis by interacting with CD4.

The correlation found between genotype (CNVs) and phenotype (VM/CCSVI) are further supported by the above reported regulatory functions in the angiogenic process. Both extragenic and intragenic CNVs with known interaction in human angiogenesis support the hypothesis that the identified genetic variants may determine disregulation in the embryological process of venous angiogenesis, possibly leading to the development of VM. VM in turn create overtime a status known as CCSVI which is strongly associated to MS.

### BIBLIOGRAPHY

1. Comabella M, et al. PLoS One 2008; 3:e3490.
2. Ramagopalan SV, et al. BMC Med Genet 2009; 10:10.
3. Chao MJ, et al. Proc Natl Acad Sci USA 2008; 105:13069-74.
4. Aulchenko YS, et al. Nat Genet. 2008; 40:1402-3.
5. Cronin S, et al. Hum Mol Genet. 2008; 17:3392-8.
6. Blauw HM, et al. Lancet Neurol. 2008; 7:319-26.
7. International Multiple Sclerosis Genetics Consortium, Hafler DA, et al. N Engl J Med. 2007; 357:851-62.
8. International Multiple Sclerosis Genetics Consortium (IMSGC). Lancet Neurol. 2008;7:567-9.
9. Zamboni P, et al. J Neurol Neurosurg Psychiatry 2009; 80:392-9.
10. Zamboni P, et al. J Neurol Sci. 2009; 282:21-7.
11. Polman CH, et al. Ann Neurol. 2005; 58:840-6.
12. Kurtzke JF. Neurology. 1983; 33:1444-52.
13. Roxburgh RH, et al. Neurology. 2005; 64:1144-51.
14. Pachner AR, Steiner I. J Neurol Sci. 2009; 278:66-70.
15. Menegatti E, Zamboni P. Curr. Neurovasc. Res. 2008; 5:260-5.
16. Agilent Technologies eArray [https://earray.chem.agilent.com/earray]
17. Bovolenta M, et al. BMC genomics. 2008, 9:572.
18. Database of Genomic Variants [http://projects.tcag.ca/variation]
19. Singh AV, Zamboni P. J Cer Blood Flow Met 2009
20. Lincoln MR, et al. Proc Natl Acad Sci USA. 2009; 106(18):7542-7.
21. Podojil JR, Miller SD. Immunol Rev. 2009; 229(1):337-55.
22. Wieder T, et al. Cell Cycle. 2008; 7(19):2974-7.

## Claims

1. A method for the *in vitro* diagnosis of a risk of developing at least one extra cranial cerebrospinal venous malformation in a patient, said method comprising detecting the global number of extragenic copy number variations (CNV) in the HLA-DRA locus of chromosome 6p21.

2. Method according to claim 1, wherein said detection is performed on a sample of genomic DNA of said patient.

3. Method according to claim 1 or claim 2, wherein said detection of said copy number variations in chromosome 6p21 is performed using a CGH method, an array CGH method, or a single nucleotide polymorphisms based array.

4. CGH array of the HLA-DRA locus of chromosome 6p21, wherein said array comprises a solid support, and a plurality of oligonucleotide probes covering the HLA-DRA locus of chromosome 6p21, wherein said probes are linked to said solid support, and wherein said plurality of oligonucleotide probes has a resolution of one probe every 160 bp of the nucleotide sequence of the HLA-DRA locus chromosome 6p21 and said oligonucleotide probes are 60mer oligonucleotides.

5. CGH array according to claim 4, wherein said plurality of oligonucleotide probes comprises 43102 probes.

6. CGH array according to claim 4 or claim 5, wherein said array has a format of 4x44K.

## Patentansprüche

1. Verfahren zur in-vitro-Diagnose eines Risikos, mindestens eine extrakranielle cerebrospinale venöse Fehlbildung zu entwickeln, bei einem Patienten, wobei das Verfahren umfasst, die globale Anzahl extragener Kopienzahlvariationen (CNV) im HLA-DRA-Locus von Chromosom 6p21 nachzuweisen.

2. Verfahren gemäß Anspruch 1, wobei der Nachweis mit einer Probe genomischer DNA des Patienten durchgeführt wird.

3. Verfahren gemäß Anspruch 1 oder Anspruch 2, wobei der Nachweis der Kopienzahlvariationen in Chromosom 6p21 unter Verwendung eines CGH-Verfahrens, eines CGH-Array-Verfahrens oder eines auf Einzelnukleotid-Polymorphismen beruhenden Arrays durchgeführt wird.

4. CGH-Array des HLA-DRA-Locus von Chromosom 6p21, wobei der Array eine feste Unterlage und eine Vielzahl von Oligonukleotidsonden umfasst, die den HLA-DRA-Locus von Chromosom 6p21 abdecken, wobei die Sonden mit der festen Unterlage verknüpft sind und wobei die Vielzahl der Oligonukleotidsonden eine Auflösung von einer Sonde pro 160 bp der Nukleotidsequenz des HLA-DRA-Locus von Chromosom 6p21 aufweist und es sich bei den Oligonukleotidsonden um 60mer-Oligonukleotide handelt.

5. CGH-Array gemäß Anspruch 4, wobei die Vielzahl von Oligonukleotidsonden 43102 Sonden umfasst.

6. CGH-Array gemäß Anspruch 4 oder Anspruch 5, wobei der Array ein Format von 4 x 44K aufweist.

## Revendications

1. Procédé de diagnostic *in vitro* d'un risque de développement d'au moins une malformation veineuse cérébrospinale extra-crânienne chez un patient, ledit procédé comprenant la détection du nombre global de variations du nombre de copies extragéniques (VNC) dans le locus HLA-DRA du chromosome 6p21.

2. Procédé selon la revendication 1, dans lequel ladite détection est réalisée sur un échantillon d'ADN génomique dudit patient.

3. Procédé selon la revendication 1 ou la revendication 2, dans lequel ladite détection desdites variations du nombre de copies dans le chromosome 6p21 est réalisée en utilisant une technique CGH, une technique CGH array ou une puce ADN basée sur des polymorphismes mononucléotidiques.

4. CGH array du locus HLA-DRA du chromosome 6p21, dans lequel ledit CGH array comprend un support solide et une pluralité de sondes oligonucléotidiques couvrant le locus HLA-DRA du chromosome 6p21, dans lequel lesdites sondes sont liées audit support solide, et dans lequel ladite pluralité de sondes oligonucléotidiques présente une résolution d'une sonde toutes les 160 pb de la séquence nucléotidique du locus HLA-DRA du chromosome 6p21 et lesdites sondes oligonucléotidiques sont des oligonucléotides 60 mer.

5. CGH array selon la revendication 4, dans lequel ladite pluralité de sondes oligonucléotidiques comprend 43102 sondes.

6. CGH array selon la revendication 4 ou la revendication 5, dans lequel ledit CGH array a un format de 4 x 44K.
